# EUROPÄISCHE PATENTANMELDUNG

(11) **EP 0 913 145 A1**
(43) Veröffentlichungstag der Anmeldung: **06.05.1999**
(21) Anmeldenummer: 98115745.6
(22) Anmeldetag: 20.08.1998
(51) Int. Cl.: A61K 7/42

(54) **Nicht fettendes Sonnenschutzmittel**

(30) Priorität: 20.08.1997 DE 29714940 U
(71) Anmelder: Stada R + D GmbH, 61118 Bad Vilbel (DE)
(72) Erfinder: Frosch, Vera, 67098 Bad Dürkheim (DE); Heppner, Andrea, 61197 Florstadt (DE); Hansen, Peter, Dr., 63303 Dreieich (DE); Schumann, Christoph, 35767 Breitscheid-Erdbach (DE)
(74) Vertreter: Maiwald, Walter, Dr. Dipl.-Chem.

(57) **Zusammenfassung**

Die Erfindung betrifft ein Sonnenschutzmittel in Form einer Lösung, das dadurch gekennzeichnet ist, daß es mindestens eine UV-filternde Verbindung gelöst in einem Lösungsmittel oder Lösungsmittelgemisch umfaßt und frei von Emulgatoren, Ölen, Fette, Wachsen und Silikonverbindungen ist, vorzugsweise umfaßt das Sonnenschutzmittel ein Lösungsmittelgemisch aus Wasser und Alkohol. Es zeichnet sich durch einen schnellen Wirkungseintritt und eine gute Hautverträglichkeit aus.

## Beschreibung

Die Erfindung betrifft ein nicht fettendes Sonnenschutzmittel in Form einer Lösung, das frei von Emulgatoren, Ölen, Fetten, Wachsen und Silikonverbindungen ist und sich durch einen besonders raschen Wirkungseintritt sowie eine ausgezeichnete Hautverträglichkeit auszeichnet.

Üblicherweise liegen Sonnenschutzprodukte in Form von Cremes, Salben, Lotionen, Gelen oder öligen Lösungen vor. Diese Produkte werden in der Regel aus geeigneten Behältern wie Tuben, Flaschen und Tiegeln, auf die Haut aufgebracht und müssen anschließend manuell auf der Hautoberfläche verteilt werden.

Zur Herstellung solcher herkömmlichen Präparate werden oftmals Emulgatoren, Öle, Fette, Wachse und/oder Silikonverbindungen eingesetzt, die verschiedenen Zwecken dienen sollen. So werden solche Hilfsmittel zur Solubilisierung der in diesen Mitteln eingesetzten UV-filternden Substanzen, zur Erzielung rückfettender Eigenschaften, zur Verbesserung der Spreitfähigkeit der Produkte, zur Herstellung von Emulsionen und/oder zur Einstellung der Viskosität verwendet. Allerdings werden diese Substanzen schon seit längerem im Zusammenhang mit Unverträglichkeitsreaktionen, wie beispielsweise der "Mallorca-Akne", kritisch diskutiert.

Neben dem Problem einer möglichen Unverträglichkeit weisen die bisher bekannten Sonnenschutzmittel eine Reihe weiterer Nachteile auf. So bedingt die erforderliche manuelle Verteilung dieser Mittel auf der Haut Verschmutzungen und Verklebungen der Hände des Anwenders und stellt darüber hinaus, insbesondere bei gründlicher Durchführung, einen zeitaufwendigen Prozeß dar.

Wegen der fettenden und verklebenden Eigenschaften solcher Mittel wird darüber hinaus eine zuverlässige Anwendung auf behaarten Bereichen der Haut äußerst erschwert und teilweise sogar unmöglich. Unabhängig hiervon wird der klebrige, fettige Effekt, den viele der herkömmlichen Produkte aufweisen, in der Regel vom Anwender als unangenehm und störend empfunden. Ebenfalls als nachteilig und unangenehm wird empfunden, daß bis zum Einziehen der Produkte ein weißer Film auf der Haut verbleibt.

Ein entscheidender Nachteil der bisher bekannten Sonnenschutzmittel liegt darin, daß die volle Wirksamkeit des UV-Schutzes in der Regel erst nach ca. 30-45 Minuten nach der Auftragung auf die Haut erreicht wird. Dieser verzögerten Wirkung ist sich der Anwender häufig nicht bewußt, so daß es in der Phase bis zum Eintritt der vollen Wirksamkeit mitunter bereits zu Schädigungen der Haut durch UV-Strahlung kommen kann, woraus sich zwangsläufig nicht zu unterschätzende gesundheitliche Risiken ergeben.

Darüber hinaus weisen bekannte Rezepturen häufig eine äußerst komplexe Zusammensetzung auf, was eine aufwendige und zeitintensive Herstellung bedingt.

Aufgabe der vorliegenden Erfindung ist es, ein Sonnenschutzmittel bereitzustellen, das die diskutierten Nachteile bekannter Produkte überwindet und sich vor allem durch einen sich rasch entfaltenden, zuverlässigen Schutz vor schädlicher UV-Strahlung auszeichnet.

Diese Aufgabe wird mittels des im unabhängigen Anspruch definierten Sonnenschutzmittels gelöst.

Die abhängigen Ansprüche definieren vorteilhafte Ausführungsformen der Erfindung.

Die erfindungsgemäßen Sonnenschutzmittel unifassen mindestens eine UV-filternde Verbindung gelöst in einem Lösungsmittel oder Lösungsmittelgemisch und sind frei von Emulgatoren, Fetten, Ölen, Wachsen und Silikonverbindungen.

Als Lösungsmittel werden bevorzugt ein- oder mehrwertige Alkohole sowie Wasser verwendet. Durch geeignete Wahl der UV-filternden Verbindung(en) können die erfindungsgemäßen Sonnenschutzmittel sowohl rein wäßrig als auch rein alkoholisch formuliert werden. Unter kosmetischen Aspekten werden jedoch solche Zubereitungen bevorzugt, die als Lösungsmittelgemisch mindestens einen Alkohol sowie Wasser in jedem beliebigen Mischungsverhältnis umfassen. Bevorzugt umfassen die erfindungsgemäßen Zusammensetzungen 5-99 Gew.-% mindestens eines ein- oder mehrwertigen Alkohols, vorzugsweise ausgewählt aus Ethanol, Isopropanol, Glycerin und Propylenglykol, mindestens eine UV-filternde Verbindung (UV-A-, UV-B- und/oder UV-A/B-Filter), in einer Menge von 1-30 Gew.-%, bevorzugt 5-25, meist bevorzugt 7,5-15 Gew.-% sowie Wasser in einer Menge von bis zu 94 Gew.-%.

Gegebenenfalls können viskositätserhöhende Substanzen wie Acrylsäure-Copolymere in einer solchen Menge enthalten sein, die keine Gelbildung bewirkt, wodurch die Haftfähigkeit der erfindungsgemäßen Zusammensetzungen auf der Haut verbessert wird.

Des weiteren können die erfindungsgemäßen Zusammensetzungen feuchtigkeitsspendende Inhaltsstoffe enthalten, wie z.B. Sorbitol, Glycerin, Panthenol, Propylenglykol und Harnstoff.

Weitere optionale Bestandteile der erfindungsgemäßen Sonnenschutzmittel können üblicherweise eingesetzte Stabilisatoren, beispielsweise Antioxidantien, Konservierungsmittel, Komplexbildner und Zusätze zur Einstellung des pH-Wertes sein.

Auch können die erfindungsgemäßen Zusammensetzungen zugelassene kosmetische Wirkstoffe, wie z.B. Vitamine, Allantoin, α-Bisabolol und Pflanzenextrakte, Duftstoffe und weitere in kosmetischen Zusammensetzungen üblicherweise verwendete Zusatzstoffe enthalten.

Das erfindungsgemäße Sonnenschutzmittel kann als solches oder mittels eines Sprühsystems auf die Haut aufgebracht werden. Eine anschließende manuelle Verteilung ist nicht oder in nur sehr geringem Umfang erforderlich. Da das Produkt keine fettenden Eigenschaften aufweist, wird es als äußerst angenehm empfunden und ermöglicht eine zuverlässige Anwendung auch auf haarigen Bereichen der Haut.

Die einfache Zusammensetzung bietet die Möglichkeit einer schnellen und kostengünstigen Herstellung des erfindungsgemäßen Sonnenschutzmittels.

Unverträglichkeitsreaktionen werden aufgrund des Fehlens von Emulgatoren, Ölen, Fetten, Wachsen und Silikonverbindungen minimiert.

In der Fachwelt galt es bisher als unumstritten, daß Sonnenschutzprodukte mit einem hohen Alkoholanteil austrocknend auf die Haut wirken und somit schlecht verträglich sind. Völlig überraschend hat sich in Testreihen mit Probanden gezeigt, daß das erfindungsgemäße Sonnenschutzmittel, auch bei hohen Alkoholgehalten ausgezeichnet hautverträglich ist.

Darüber hinaus zeichnet sich das erfindungsgemäße Sonnenschutzmittel durch eine rasche Wirkung und das Erreichen besonders hoher Lichtschutzfaktoren binnen kurzer Zeit nach der Aufbringung auf die Haut aus. So erreicht das erfindungsgemäße Mittel bereits 5 Minuten nach der Auftragung einen Lichtschutzfaktor, der ungefähr 90%, bevorzugt über 90% und besonders bevorzugt über 95%, des angestrebten, durch die jeweilige Konzentration und/oder Kombination der enthaltenen UV-filternden Substanzen einstellbaren Lichtschutzfaktorwertes beträgt.

Die vorliegende Erfindung wird, ohne sie dadurch beschränken zu wollen, durch die folgenden Beispiele verdeutlicht. Alle Angaben beziehen sich auf Gewichtsprozent.

### Beispiel 1

| | % |
|---|---|
| Alkohol, vergällt | 81,0 |
| 3-(4-Methylbenzyliden)-campher | 3,0 |
| 4-Methoxyzimtsäure-isoamylester | 3,0 |
| 2-Cyano-3,3-diphenylacrylsäure-2'-ethylhexylester | |
| | 3,0 |
| 4-tert.Butyl-4-methoxydibenzoylmethan | 0,5 |
| α-Bisabolol nat. | 0,1 |
| DL-α-Tocopherol | 1,0 |
| Wasser | 8,4 |

Die Herstellung der Rezeptur erfolgt nach folgendem Verfahren:
In einem ersten Schritt werden die UV-filternden Verbindungen 4-Methoxyzimtsäureisoamylester und 2-Cyano-3,3-diphenylacrylsäure-2'-ethylhexylester miteinander vermischt. Anschließend werden 3-(4-Methylbenzyliden)-campher und 4-tert.Butyl-4-methoxydibenzoylmethan unter Erwärmen hinzugegeben und gelöst. Nachfolgend werden zunächst α-Bisabolol nat. und DL-α-Tocopherol und anschließend Alkohol,vergällt, hinzugefügt und das Gemisch unter Rühren in Lösung gebracht. Schließlich wird Wasser unter Rühren hinzugefügt und die Zusammensetzung anschließend nochmals für weitere 10 Minuten gerührt.

In einer Testreihe mit Probanden erreichte die Zuammensetzung bereits 5 Minuten nach der Auftragung auf die Haut einen Lichtschutzfaktor von ungefähr 14,7, der weiter auf ca. 14,9 nach 10 Minuten und auf ca. 15,0 (maximaler Lichtschutzfaktor) nach 15 Minuten anstieg.

### Beispiel 2

| | % |
|---|---|
| Alkohol, vergällt | 12 |
| 2-Phenylbenzimidazol-5-Sulfonsäure | 5,0 |
| Benzophenon-4 | 5,0 |
| Allantoin | 0,2 |
| Glycerin DAB 10 | 4,0 |
| Acrylsäure-Copolymer | 0,1 |
| Na-EDTA-Dinatrium | 0,005 |
| Triethanolamin | 7,1 |
| Wasser | 66,595 |

Die Herstellung eines 1 kg-Ansatzes erfolgte in folgenden Verfahrensschritten:
Zunächst wird das Acrylsäure-Copolymer (Pemulen TR1 von BFGoodrich Company) unter Einsatz eines Ultra-Turrax in 200 g Wasser dispergiert. Anschließend werden 120 g Alkohol, vergällt, hinzugegeben und der Ansatz 30 Minuten quellen gelassen. Separat werden die UV-filternden Verbindungen 2-Phenylbenzimidazol-5-Sulfonsäure und Benzophenon-4 in einem zweiten Ansatz in 265,95 g Wasser aufgeschlämmt, anschließend Triethanolamin hinzugegeben und der Ansatz unter Verwendung eines Ultra-Turrax gelöst. In einem dritten Ansatz werden Glycerin, Allantoin und EDTA-Dinatrium in 200 g Wasser gelöst. Anschließend werden die erste und die dritte Phase vereinigt und kurz homogenisiert. Schließlich wird die zweite Phase unter Homogenisieren hinzugegeben, wobei die gesamte Homogenisierzeit ca. 1 Minute umfaßt, und die Zusammensetzung abschließend 10 Minuten gerührt.

In einer Testreihe mit Probanden erreichte die Zuammensetzung bereits 5 Minuten nach der Auftragung auf die Haut einen Lichtschutzfaktor von ungefähr 15,2, der weiter auf ca. 15,5 nach 10 Minuten und auf ca. 15,7 (maximaler Lichtschutzfaktor) nach 15 Minuten anstieg.

Im Vergleich hierzu zeigte sich bei einem in üblicher Weise formulierten Gelprodukt, das dieselben Filter in denselben Konzentrationen enthielt, wie das zuvor beschriebene Produkt in Form einer Lösung, nach einer Einwirkzeit von 5 Minuten ein Lichtschutzfaktor von ca. 12,6, der nach 10 Minuten auf ca. 13,5 und erst nach 45 Minuten auf den maximalen Lichtschutzfaktor von 17,3 anstieg.

Hieraus wird deutlich, daß mit den erfindungsgemäßen Lichtschutzzubereitungen ein sehr viel schnellerer Wirkungseintritt als bei handelsüblichen Präparaten realisiert werden kann.

### Beispiel 3

In einer klinischen Untersuchung wurde das Produkt gemäß Beispiel 1 mittels des sogenannten "Patch-Tests" geprüft. Mit diesem Patch-Test können dermatologische und kosmetische Produkte auf ihr irritatives Potential untersucht werden.

Alle Untersuchungen erfolgten nach GLP-Richtlinien und entsprechend den Empfehlungen der COLIPA Arbeitsgruppe (Walter A.P. et al: Test Guidelines for Assessment of Skin Compatibility of Cosmetic Finished Products in Ma. Food and Chemical Toxicology 34, 1996, 651-660).

Die Untersuchung erfolgte an 30 Testpersonen (19 Hautgesunde, 2 Atopiker, 2 Allergiker, 7 Personen mit empfindlicher Haut) im Alter von 18-50 Jahren. Während der Testung verzichteten die Probanden in dem Testareal auf die Benutzung von Externa.

### Durchführung

50µl des Produkts nach Beispiel 1 wurden unverdünnt auf den Rücken der Probanden mit Hilfe von quadratischen Kunststoffkammern (Kantenlänge: 1cm) für 48 h unter Okklusion appliziert.

Als Positivkontrolle diente der Modellschadstoff Natriumlaurylsulfat (SDS) in zwei Konzentrationen (0,2% und 1%). Als negative Kontrolle diente Wasser.

Die Bewertung der Testreaktionen erfolgt nach 48 h und 72 h nach dem modifizierten Draize-Test:
- Erythem: 0: kein E., 1: leichtes E., 2: deutliches E., 3: ausgeprägtes E., 4: starkes E.
- Fissur: 0: keine F., 1: minimale F., 2: deutlich wahrnehmb.F. 3: ausgeprägte F., 4: Ulcerationen
- Schuppung: 0: keine Sch., 1: minimale Sch., 2: mäßige Sch., 3: deutliche Sch., 4: geschlossene Schuppenkruste

Die Tests haben ergeben, daß unter den Testbedingungen die 1%ige SDS-Lösung bei 15 Testpersonen zu einer positiven Reaktion führte. Die 0,2%ige SDS-Lösung zeigte bei 9 Testpersonen eine Reaktion. Die negative Kontrolle zeigte wie erwartet bei keiner Person eine Reaktion.

Auch bei den Tests unter Verwendung des Sonnenschutzmittels gemäß Beispiel 1 zeigte sich bei keiner Versuchsperson eine Reaktion, so daß dieses hinsichtlich einer eventuell hautreizenden Wirkung als unbedenklich einzustufen ist.

## Patentansprüche

1. Sonnenschutzmittel in Form einer Lösung,
**dadurch gekennzeichnet,** daß es mindestens eine UV-filternde Verbindung gelöst in einem Lösungsmittel oder Lösungsmittelgemisch umfaßt und frei von Emulgatoren, Ölen, Fette, Wachsen und Silikonverbindungen ist.

2. Mittel nach Anspruch 1,
**dadurch gekennzeichnet,** daß es als Lösungsmittel Wasser umfaßt.

3. Mittel nach Anspruch 1,
**dadurch gekennzeichnet,** daß es als Lösungsmittel mindestens einen ein- oder mehrwertigen Alkohol umfaßt.

4. Mittel nach einem der vorstehenden Ansprüche,
**dadurch gekennzeichnet,** daß es 5-95 Gew.-% mindestens eines ein- oder mehrwertigen Alkohols sowie 1-94 Gew.-% Wasser umfaßt.

5. Mittel nach einem der vorstehenden Ansprüche,
**dadurch gekennzeichnet,** daß es 10-90 Gew.-%, bevorzugt 20-85 Gew.-%, meist bevorzugt 30-85 Gew.-%, eines ein- oder mehrwertigen Alkohols umfaßt.

6. Mittel nach einem der vorstehenden Ansprüche,
**dadurch gekennzeichnet,** daß es zusätzlich mindestens eine feuchtigkeitsspendende Verbindung enthält.

7. Mittel nach Anspruch 6,
**dadurch gekennzeichnet,** daß als feuchtigkeitsspendende Verbindung Sorbitol, Glycerin, Panthenol, Propylenglykol und/oder Harnstoff umfaßt.

8. Mittel nach einem der vorstehenden Ansprüche,
**dadurch gekennzeichnet,** daß es zusätzlich übliche Zusatzstoffe, wie Stabilisatoren, Antioxidantien, Konservierungsmittel, Komplexbildner und/oder Zusätze zur Einstellung des pH-Wertes umfaßt.

9. Mittel nach einem der vorstehenden Ansprüche,
**dadurch gekennzeichnet,** daß es zusätzlich mindestens einen kosmetischen Wirkstoff umfaßt.

10. Mittel nach Anspruch 9,
**dadurch gekennzeichnet,** daß es als kosmetischen Wirkstoff ein oder mehrere Vitamine, Allantoin, α-Bisabolol und/oder einen oder mehrere Pflanzenextrakte enthält.

11. Mittel nach einem der vorstehenden Ansprüche,
**dadurch gekennzeichnet,** daß es zusätzlich mindestens einen Duftstoff enthält.

12. Mittel nach einem der vorstehenden Ansprüche,
**dadurch gekennzeichnet,** daß es zusätzlich mindestens ein viskositätserhöhendes Mittel enthält.

13. Mittel nach dem der vorstehenden Ansprüche,
**dadurch gekennzeichnet,** daß es
75-85 Gew.-% mindestens eines Alkohols
5-10 Gew.-% Wasser
5-15 Gew.-% mindestens einer UV-filternden Verbindung und
0,5-1,5 Gew.-% eines viskositätserhöhenden Mittels umfaßt.

14. Mittel nach Anspruch 13,
**dadurch gekennzeichnet,** daß es
81 Gew.-% Alkohol
3,0 Gew.-% 3-(4-Methylbenzyliden)-campher
3,0 Gew.-% 4-Methoxyzimtsäure-isoamylester
3,0 Gew.-% 2-Cyano-3,3-diphenylacrylsäure-2'-ethylhexylester
0,5 Gew.-% 4-tert.Butyl-4-methoxydibenzoylmethan
0,1 Gew.-% α-Bisabolol
1,0 Gew.-% DL-α-Tocopherol
8,4 Gew.-% Wasser umfaßt.

15. Mittel nach einem der vorstehenden Ansprüche,
**dadurch gekennzeichnet,** daß es mittels eines Sprühsystems auf die Haut aufgebracht wird.
